# EUROPEAN PATENT SPECIFICATION

(11) **EP 0 656 908 B1**
(45) Date of publication and mention of the grant of the patent: **20.01.1999**
(21) Application number: 93919457.7
(22) Date of filing: 20.08.1993
(51) Int. Cl.: C07K 16/22, C07K 14/65, A61K 38/18

(54) **USE OF SPECIFIC BINDING MOLECULES IN POTENTIATING IGF-I ACTIVITY**
VERWENDUNG VON SPEZIFISCHEN MOLEKÜLEN ZUR STEIGERUNG DER IGF-1 AKTIVITÄT
UTILISATION DE MOLECULES SPECIFIQUES POUR POTENTIALISER L'ACTIVITE DU FACTEUR DE CROISSANCE INSULINOIDE I

(30) Priority: 20.08.1992 GB 9217696
(43) Date of publication of application: 14.06.1995
(73) Proprietor: Biotechnology and Biological Sciences Research Council, Swindon SN2 1UH (GB)
(72) Inventor: PELL, Jennifer Margaret, Ickleton, Cambs CB10 1SR (GB); BATES, Peter Charles, Newport, Saffron Walden CB11 3QF (GB); STEWART, Claire Elisabeth Hamilton, St Louis, MO 63108 (US)
(74) Representative: Sheard, Andrew Gregory
(86) International application number: GB9301774
(87) International publication number: WO9404569

(56) References cited:
- GENE. vol. 58, no. 1 , 1987 , AMSTERDAM NL pages 87 - 97 B. LÖWENADLER ET AL. 'A GENE FUSION SYSTEM FOR GENERATING ANTIBODIES AGAINST SHORT PEPTIDES.'
- PROCEEDINGS OF THE NATIONAL ACADEMY OF SCIENCES OF USA. vol. 84 , May 1987 , WASHINGTON US pages 3254 - 3258 R.G. ELGIN ET AL. 'AN INSULIN-LIKE GROWTH FACTOR (IGF) BINDING PROTEIN ENHANCES THE BIOLOGIC RESPONSE TO IGF-I.' cited in the application
- CHEMICAL ABSTRACTS, vol. 114, no. 21, 27 May 1991, Columbus, Ohio, US; abstract no. 200433c, G.S.G. SPENCER ET AL. 'PASSIVE IMMUNIZATION AGAINST INSULIN-LIKE GROWTH FACTOR-1 DOES NOT INHIBIT GROWTH HORMONE-STIMULATED GROWTH OF DWARF RATS.' page 175 ; cited in the application & ENDOCRINOLOGY (BALTIMORE) vol. 128, no. 4 , 1991 pages 2103 - 2109
- CHEMICAL ABSTRACTS, vol. 115, no. 19, 11 November 1991, Columbus, Ohio, US; abstract no. 199067y, J.M. PELL ET AL. 'ACTIVE IMMUNIZATION WITH A SYNTHETIC PEPTIDE REGION OF GROWTH HORMONE: INCREASED LEAN TISSUE GROWTH.' page 157 ; & J. ENDOCRINOL. vol. 131, no. 1 , 1991 pages R1 - R4

## Description

This invention relates to specific binding molecules, means for generating them, and their use to potentiate or enhance one or more biological activities of insulin-like growth Factor I.

Insulin-like growth factor-I (IGF-I) is a potent peptide hormone (molecular weight of approximately 7,500 kDa) which is thought to mediate many of the anabolic actions of growth hormone (GH) as well as having independent actions to stimulate cell hyperplasia, hypertrophy and differentiation (Daughaday and Rotwein, *Endocrine Reviews* 10 68-92 (1989)). IGF-I is synthesised by many tissues in the body and may therefore exert local autocrine/ paracrine actions; in addition, the liver secretes large amounts of IGF-I into the blood where it can have widespread endocrine actions on peripheral tissues. IGF-I usually binds with high affinity to type 1 receptors, which are thought to mediate most of its actions. However, it can also bind weakly to insulin and type 2/mannose-6-phosphate receptors.

Only very small amounts of IGF-I are found as free peptide in *vivo.* Instead, it is bound to binding proteins (IGFBPs) of which six high affinity proteins have been characterised so far (Sara and Hall, *Physiological Reviews* **70** 591-614 (1990)). The precise function of this complex arrangement of IGFBPs with IGF-I is unknown at present but several options have been suggested: neutralisation of the "insulin like" actions of IGF-I, inhibition of general IGF-I activity, transport of IGF-I from blood to tissues or from one cell type to another, targeting of IGF-I towards tissue receptors, maintenance of the IGF-I pool. Most experiments to date, both *in vivo* and *in vitro,* have indicated that IGFBPs are inhibitory for IGF-I action, although some reports have demonstrated enhancement of IGF-I activity *in vitro* (Elgin *et al, Proceedings of the National Academy of Sciences of the USA,* **84** 3254-3258 (1987); and Conover, *Endocrinology* **130** 3191-3199 (1992)). This apparent potentiation of IGF-I action is thought to be due to the adherence of the IGFBPs to cell surfaces thus presenting IGF-I to its receptor; decreased affinity of the BPs for IGF-I may also be involved, allowing receptors to compete more effectively for IGF-I.

WO-A-8908667 and WO-A-8909268 both disclose the production of recombinant IGFBPs; the former additionally discloses enhancement of IGF-I activity by the recombinant protein.

Some structural properties of IGF-I have been identified. IGFBPs generally bind to residues 1-18 and 49-51 of IGF-I. Des(1-3) IGF-I and long-R IGF-I, which are IGF-I analogues that bind poorly to IGFBPs, exhibit increased activity compared with IGF-I alone both *in vivo* and *in vitro* (Tomas et *al, Biochemical Journal* **282** 91-97 (1992)). Type 1 receptor activity and binding is associated with residues 22-37 (equivalent to the C-peptide region of pro-insulin). Type 2 receptor binding is in the region of residues 49-58. The function of the C-terminal D-loop is unknown.

Polyclonal and monoclonal antibodies against IGF-I are known. EP-A-0292656, for example, discloses monoclonal antibodies against IGF-I and their use in an immunometric assay for IGF-I. As far as the effect of such antibodies on IGF-I activity is concerned, all IGF-I binding antibodies studied to date (monoclonal or polyclonal) have either inhibited IGF-I activity or had no demonstrable effect on IGF-I action. For example, studies demonstrating inhibitory action of anti-IGF-I antibodies include the following:
Schlechter *et al, American Journal of Physiology* **250** E231-E235 (1986);
   Ooi and Herington, *Biochemical and Biophysical Research Communications* **156** 783-791 (1988);
   Gluckman, Disclosure at the Second International Conference on IGFs at San Francisco on January 12th to 16th 1991 and submitted to *J. Endocrinol* (1991); Mondschein *et al, Biology of Reproduction* **40** 79-85 (1989);
   Morrell *et al, J. Molec. Endocrinol.* **2** 201-206 (1989) and
   Bicsak *et al, Ehdocrinology* **126** 2184-2189 (1990).

The following studies demonstrate no apparent action of anti-IGF-I antibodies:
Kerr *et al, J. Endocrinol.* **124** 403-415 (1990) and Spencer *et al, Endocrinology* **128** 2103-2109 (1991)

To complete this brief survey of the known literature, Tamura *et al, J. Endocrinol.* **125** 327-335 (1990) did not draw any conclusions on the action of specific anti-IGF-I antibodies studied.

It has now been found that it is in fact possible to enhance the in *vivo* activity of endogenous or exogenous IGF-I by antibody binding, or by the binding of other specific binding molecules. Furthermore, the fact that the observed enhancement is not confined to natural IGF binding proteins shows that there is apparently a different mechanism at work from that previously proposed in the observations on IGFBPs.

According to a first aspect of the invention, there is provided a specific binding molecule, other than a natural IGF binding protein, which is capable of binding to insulin-like growth factor-I (IGF-I) and which is capable of enhancing a biological activity of IGF-I.

The term "specific binding molecule" as used in this specification includes natural antibodies (whether polyclonal or monoclonal), antibody fragments and modified and chimeric antibodies (including humanised antibodies), all of which have antigen (in this case IGF-I) binding capability. Antibodies and antibody-derived molecules will be preferred, not least for their ease of availability and/or preparation and their familiarity to those skilled in the art. Polyclonal and monoclonal natural antibodies may be chief amongst those preferred, but humanised antibodies (for example as taught by Winter and Milstein *(Nature* **349** 293-299 (1991)) may be as suitable, or even optimal, in certain circumstances.

The specific binding molecule, whatever its nature, is capable of enhancing at least one biological activity of IGF-I. Such an activity may, but will not necessarily be an IGF-I-mediated anabolic action of growth hormone.

IGF-I has been shown to have wide reaching actions and therefore this invention could have applications for animal growth and biotechnology, veterinary practice and clinical medicine. IGF-I can stimulate or improve the processes listed below and therefore enhancement of IGF-I activity has similar applications. In particular, therefore, the (or one of the) biological activities of IGF-I may include:
Increasing whole body and muscle growth rate in normal and hypopituitary animals (Schoenle *et al, Nature* **296** 252-253 (1982), and Hizuka *et al, European Journal of Pharmacology* **125** 143-146 (1986));
Protection of body weight and nitrogen loss during catabolic states (such as fasting, nitrogen restriction, elevated corticosteroid levels and/or diabetes) (Ballard *et al,* "Effects of IGF-I and IGF analogues on growth during catabolic states in rats" In: Modern Concepts of Insulin-like Growth Factors, pp 617-627. Ed., Spencer, E.M. Elsevier: New York (1991));
Kidney regeneration (Flyvbjerg *et al,* "Kidney IGF-I accumulation occurs in four different conditions with rapid initial kidney growth in rats" In: Modern Concepts of Insulin-like Growth Factors, pp 207-217. Ed., Spencer, E.M. Elsevier: New York (1991));
Nerve regeneration (Komoly *et al, Proceedings of the National Academy of Sciences USA* **89** 1894-1898 (1992));
Hypoxia (Gluckman *et al, Biochemical and Biophysical Research Communications* **182** 593-599 (1992));
Wound healing (Mueller *et al,* "The role of IGF-I and IGFBP-3 in wound healing" In: Modern Concepts of Insulin-like Growth Factors, pp 185-192. Ed., Spencer, E.M. Elsevier: New York (1991)); and Jennische *et al,* "Local expression of somatomedins during tissue growth and regeneration" In: The Insulin-like Growth Factors, Structure and Biological Functions. Ed. Schofield, P. CRS: Oxford (in press) (1991));
Cardiac regeneration (Florini *et al,* "Proteins induced by the IGFs in skeletal, smooth and cardiac muscle: implications for cardiovascular diseases" In: Modern Concepts of Insulin-like Growth Factors, pp 487-503 Ed., Spencer, E.M. Elsevier: New York (1991));
Cancer cachexia (Ng *et al, American Journal of Physiology* **262**(3) R426-R431 (1992));
Angiogenesis (Nakaohayashi *et al, Atherosclerosis 92* 141-149 (1992));
Regeneration of the gastrointestinal tract (Vanderhoof *et al, Gastroenterology* **102** 1949-1956 (1992));
Stimulation of mammary function (Peri *et al, Cell Biology International Reports* **16** 359-368 (1992));
IGF-I-dependent actions of GH (such as, usefulness in metabolic stress, age-related decreases in GH activity and adult GH deficiency) (M. Keliman, *Journal of the American Geriatrics Society* **39** 295-307 (1991); F.E. Kaiser, *Geriatrics* **47** 85 (1992); Chwals and Bistrian, *Critical Care Medicine* **19** 1317-1322 (1991); and Sonksen *et al, Acta Paediatrica* S379 139-146 (1991));
Usefulness in maturity-onset diabetes (Schalch *et al,* "Short-term metabolic effects of recombinant human insulin-like growth factor I (rhIGF-I) in type II diabetes mellitus" In: Modern Concepts of Insulin-like Growth Factors, pp 705-713 Ed., Spencer, E.M. Elsevier: New York (1991)); and/or
Usefulness in specific IGF-I deficiency (Laron dwarfism) (Laron *et al, Lancet* **339**(8804) 1258-1261 (1992)).

Neither the above nor any other medical utilities for specific binding molecules for IGF-I (including IGF binding proteins) has been suggested.

The complete amino acid sequence of mature, human IGF-I is as follows:

1          11          21          31
GPETLCGAEL  VDALQFVCGD  RGFYFNKPTG  YGSSSRRAPQ

41          51          61
TGIVDECCFR  SCDLRRLEMY  CAPLKPAKSA.

Among the regions of IGF-I to which specific binding molecules in accordance with the invention may bind is included at least part of the region encompassing residues 1 to 17, and particularly 11 to 17, as numbered above from the N-terminus of mature IGF-I. Other preferred regions include those encompassing residues 18 to 21, 45 to 53, 54 to 60 and, especially, 36 to 44. Further preferred regions include those encompassing 22 to 37 and 59 to 70. The specific binding molecules need not bind to all of such a preferred region; however, a peptide fragment of some or all of this (or any other) preferred region of IGF-I may bind, for example competitively with IGF-I itself to specific binding molecules within the invention. It should be stressed that other regions of IGF-I may also be preferred as epitopes for molecules of the invention, while some for preference should be avoided. It is much preferred, though, that the specific binding molecules of the invention do not bind to other, or other significant, proteins,especially IGF-II or insulin, with both of which IGF-I shares some characteristics.

It has been stated above that antibodies are the specific binding molecules preferred for use in the invention. One particularly convenient way of ensuring a plentiful supply of antibodies is to generate them in *situ.* This can be achieved by administering to a (generally human) subject an appropriate antigenic molecule.

According to a third aspect of the invention, there is therefore provided an antigenic molecule which is capable, upon (generally parenteral) administration to a subject, of causing the generation of antibodies, wherein the antibodies are capable of binding to insulin-like growth factor-I (IGF-I) and of enhancing a biological activity of IGF-I.

Suitable antigenic molecules may (but will not necessarily) be based on peptide fragments, for example those corresponding to a region of IGF-I to which binding is sought. On this basis, a suitable antigenic molecule may be based on a fragment encompassing some or (preferably) all of residues identified above as being preferred (and possibly no significant other fragment sequence of IGF-I). Whether or not specifically so based, a preferred antigenic molecule in accordance with this aspect of the invention causes antibodies to be raised against at least some of the above-identified residues of IGF-I.

Short peptides will not as a rule be antigenic in their own right, although they can confer epitope specificity. They may be rendered antigenic by a variety of means, such as those disclosed in WO-A-8900166. Essentially, they may be coupled to carrier molecules, such as albumin, by coupling agents (such as glutaraldehyde), to form a suitable antigen molecule. Of course, equivalent and other antigenic molecules containing the peptide fragment of interest may be synthesised by recombinant DNA technology, or even chemically.

Suitable short peptides on which antigenic molecules may be based include, but are not limited to, hexamers, heptamers and octamers. Hexameric peptides derived from the regions of IGF-I identified as preferred include:

(those including at least four residues from, and
preferably wholly within, the 1-17 region,
GPETLCGAELVDALQFV)

GPETLC
 PETLCG
  ETLCGA
   TLCGAE
    LCGAEL
     CGAELV
      GAELVD
       AELVDA
        ELVDAL
         LVDALQ
          VDALQF
           DALQFV
            ALQFVC
             LQFVCG

(those encompassing the 18-21 region, CGDR)

FVCGDR
 VCGDRG
  CGDRGF

(those including at least four residues from, and
preferably wholly within, the 22-37 region,

GFYFNKPTGYGSSSRR)
DRGFYF

RGFYFN
GFYFNK
FYFNKP
YFNKPT
FNKPTG
NKPTGY
KPTGYG
PTGYGS
TGYGSS
GYGSSS
YGSSSR
GSSSRR
SSSRRA
SSRRAP

(those including at least four residues from, and
preferably wholly within, the preferred 36-44
region, RRAPQTGIV)
SSRRAP
SRRAPQ
RRAPQT
RAPQTG
APQTGI
PQTGIV
QTGIVD
TGIVDE

(those including at least four residues from, and
preferably wholly within, the 45-53 region,
DECCFRSCD'
IVDECC
VDECCF
DECCFR
ECCFRS
CCFRSC
CFRSCD
FRSCDL
RSCDLR

(those including at least four residues from, and
preferably wholly within, the 54-60 region,
LRRLEMY)
CDLRRL
DLRRLE
LRRLEM
RRLEMY
RLEMYC
LEMYCA

Heptameric peptides derived from the regions of IGF-I identified as preferred include:

(those including at least four residues from, and
preferably wholly within. the 1-17 region,
GPETLCGAELVDALQFV)
GPETLCG
PETLCGA
ETLCGAE
TLCGAEL
LCGAELV
CGAELVD
GAELVDA
AELVDAL
ELVDALQ
LVDALQF
VDALQFV
DALQFVC
ALQFVCG
LQFVCGD

(encompassing the 18-21 region, CGDR)

QFVCGDR
FVCGDRG
VCGDRGF
CGDRGFY

(those including at least four residues from, and
preferably wholly within, the 22-37 region,
GFYFNKPTGYGSSSRR)
GDRGFYF
DRGFYFN
RGFYFNK
GFYFNKP
FYFNKPT
YFNKPTG
FNKPTGY
NKPTGYG
KPTGYGS
PTGYGSS
TGYGSSS
GYGSSSR
YGSSSRR
GSSSRRA
SSSRRAP
SSRRAPQ

(those including at least four residues from, and
preferably wholly within, the preferred 36-44
region, RRAPQTGIV)

SSSRRAP
SSRRAPQ
SRRAPQT
RRAPQTG
RAPQTGI
APQTGIV
PQTGIVD
QTGIVDE
TGIVDEC

(those including at least four residues from, and
preferably wholly within, the 45-53 region,
DECCFRSCD)
GIVDECC
IVDECCF
VDECCFR
DECCFRS
ECCFRSC
CCFRSCD
CFRSCDL
FRSCDLR
RSCDLRR

(those including at least four residues from, and
preferably wholly within, the 54-60 region,
LRRLEMY)
SCDLRRL
CDLRRLE
DLRRLEM
LRRLEMY
RRLEMYC
RLEMYCA
LEMYCAP

Octameric peptides derived from the regions of IGF-I identified as preferred include:

(those including at least four residues from, and
preferably wholly within, the 1-17 region,
GPETLCGAELVDALQFV)
GPETLCGA
 PETLCGAE
  ETLCGAEL
   TLCGAELV
    LCGAELVD
     CGAELVDA
      GAELVDAL
       AELVDALQ
        ELVDALQF
         LVDALQFV
          VDALQFVC

DALQFVCG
ALQFVCGD
LQFVCGD

(encompassing the 18-21 region, CGDR)

LQFVCGDR
QFVCGDRG
FVCGDRGF
VCGDRGFY
CGDRGFYF

(those including at least four residues from, and
preferably wholly within, the 22-37 region,
GFYFNKPTGYGSSSRR)
CGDRGFYF
GDRGFYFN
DRGFYFNK
RGFYFNKP
GFYFNKPT
FYFNKPTG
YFNKPTGY
FNKPTGYG
NKPTGYGS
KPTGYGSS
PTGYGSSS
TGYGSSSR
GYGSSSRR
YGSSSRRA
GSSSRRAP
SSSRRAPQ
SSRRAPQT

(those including at least four residues from, and
preferably wholly within, the preferred 36-44
region, RRAPQTGIV)

GSSSRRAP
SSSRRAPQ
SSRRAPQT
SRRAPQTG
RRAPQTGI
RAPQTGIV
APQTGIVD
PQTGIVDE
QTGIVDEC
TGIVDECC

(those including at least four residues from, and
preferably wholly within, the 45-53 region,
DECCFRSCD)

TGIVDECC

GIVDECCF
IVDECCFR
VDECCFRS
DECCFRSC
ECCFRSCD
CCFRSCDL
CFRSCDLR
FRSCDLRR
RSCDLRRL

(those including at least four residues from, and
preferably the whole of, the 54-60 region,
LRRLEMY)
RSCDLRRL
SCDLRRLE
CDLRRLEM
DLRRLEMY
LRRLEMYC
RRLEMYCA
RLEMYCAP
LEMYCAPL

Both specific binding molecules and antigenic molecules in accordance with the invention will usually be intended to be administered parenterally, whether by injection, infusion or implantation. They will therefore generally be formulated as sterile preparations with a suitable carrier (such as water for injections or phosphate-buffered saline). Antigenic molecules within the invention may be formulated with a suitable adjuvant (such as alhydrogel) to boost antigenicity.

The invention may be used to enhance the activity of IGF-I, whether endogenous in the subject or exogenously administered. Formulations of specific binding molecules, or antigenic molecules, in accordance with the invention and IGF-I itself are therefore contemplated. Further, the specific binding molecules or antigenic molecules of the invention may be administered separately, simultaneously or sequentially with IGF-I.

The invention is useful in a method of treating or preventing conditions in which IGF-I is useful, particularly those mentioned earlier. The invention therefore relates to the use of molecules in accordance with the invention in the manufacture of a medicament for treating or preventing conditions in which IGF-I is useful.

The invention will now be illustrated by the following examples. The examples refer to the accompanying drawings, in which:
FIGURE 1a shows the binding of anti-IGF-I antiserum to IGF-I;
FIGURE 1b shows the binding of anti-IGF-I antiserum to IGF-I peptide 1-17;
FIGURE 1c shows the results of peptide scanning to identify certain IGF-I binding regions recognised by anti-IGF-I antiserum;
FIGURE 2 shows the effect of anti-IGF-I antiserum on whole body weight gain in dwarf mice;
FIGURE 3 shows the effect of anti-IGF-I antiserum on liver weight gain in dwarf mice;
FIGURE 4 shows the effect of anti-IGF-I antiserum on whole body weight gain in dwarf rats;
FIGURE 5 shows the effect of anti-IGF-I antiserum on gastrocnemius muscle weight in dwarf rats;
FIGURE 6 shows the effect of semi-purified anti-IGF-I immunoglobulin on whole body weight gain in dwarf rats; and
FIGURE 7 shows the effect of semi-purified anti-IGF-I immunoglobulin on total leg muscle (gastrocnemius, plantaris and soleus) weight in dwarf rats.

### Antibody Production

Polyclonal antibodies were raised against recombinant human IGF-I using adult wether Poll Dorset sheep. IGF-I was conjugated to human α-globulin using glutaraldehyde as follows: IGF-I (2 mg) was dissolved in 1.2 ml phosphate buffered saline (PBS, 100 mM sodium phosphate, pH 7.2). Human α-globulin (0.4 ml of a 5 mg/ml solution in PBS) and glutaraldehyde (0.4 ml of 0.4% grade I glutaraldehyde solution in water) were added and the conjugation allowed to proceed at room temperature for 20 min at which point 1.2 ml PBS were added. The mixture was immediately mixed with 6.4 ml Freund's adjuvant (complete for the prime immunisation and incomplete for subsequent boosts) using a POLYTRON homogeniser. (The word POLYTRON is a trade mark.) 2 ml were injected into sheep immediately (as 2 x 0.5 ml subcutaneously and 2 x 0.5 ml intramuscularly). The sheep were challenged every two months for a period of one year.

The titre of the antiserum was determined using standard ELISA techniques. ELISA plates (96 well) were coated for 1 h at 37°C and overnight at 4°C with a 2 µg/ml solution of IGF-I in carbonate buffer (100 mM sodium carbonate, pH 9.6). The plates were then rinsed three times with PBS containing 0.1% Tween 20 and blocked for 2 h at 37°C with 0.5% bovine serum albumin (BSA) in PBS. The wells were emptied of blocking reagent and antiserum (100 µl of appropriate dilutions in PBS containing 0.1% Tween-20 and 0.5% BSA was added. The plates were incubated for 1 h at 37°C and were then washed three times using PBS containing 0.1% Tween-20. Biotinylated anti-sheep immunoglobulin (commercial preparation from Amersham International plc, Amersham, UK) was added (100 µl of a 1:1000 dilution in the PBS/Tween-20/BSA buffer) and incubated for 1 h at 37°C, after which time the plates were washed three times in PBS/Tween-20 buffer. Subsequently, streptavidin biotin horseradish peroxidase (100 µl of a 1:1000 dilution in PBS/Tween 20/BSA) was added and incubated at 37°C for 1 h followed by washing three times in PBS/Tween-20 buffer. Substrate reagent was then added (100 µl of 0.55 mg/ml 2,2'-azino-bis(3-ethylbenzthiazoline-6-sulphonic acid, diammonium salt in 100 mM citrate buffered saline, pH 4.3 containing 1.2 µl 30% hydrogen peroxide per 20 ml). The optical density of the wells was measured at a wavelength of 405 nm at appropriate time intervals (5 to 20 min after addition of substrate reagent).

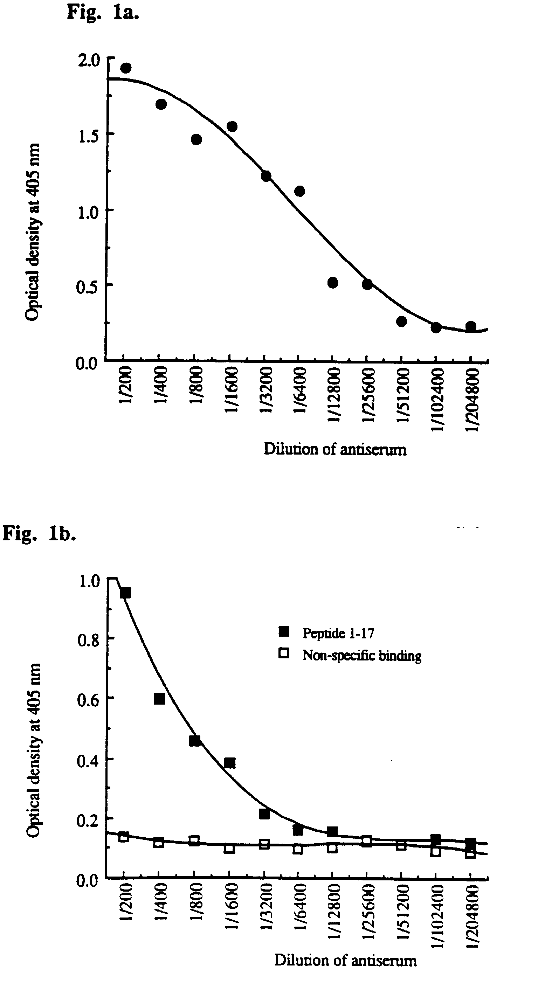

The anti-IGF-I antiserum was prepared by taking blood from sheep 50, allowing the blood to clot at 4°C for 4 h, centrifuging at 2,500 g for 30 min and harvesting the supernatant serum. Titres for IGF-I of 1 in 5,000 (for 50% binding) were determined by ELISA (Figure la). The antibody binds to IGF-I in the region of residues 1-17 determined by ELISA as described above when peptide 1-17 was used to coat the plates in place of IGF-I (Figure 1b); the antibody could also bind to other epitopes on IGF-I. The antibody exhibited negligible binding to insulin or insulin-like growth factor-II.

### Example 1: The effects of anti-IGF-I antiserum on growth in dwarf mice

Homozygous Snell dwarf mice have a genetic lesion resulting in almost negligible growth hormone, prolactin and thyroid stimulating hormone secretion by the pituitary gland and therefore almost negligible circulating IGF-I concentrations; tissue concentrations are unknown. The mice therefore provide a very sensitive model for the investigation of IGF-I activity. Homozygous dwarf mice were bred from a heterozygous colony at the Institute of Animal Physiology and Genetics Research and nine-week old mice (males and females) were randomly allocated to one of five treatment groups (n=6 per group):

| Group | Treatment |
|---|---|
| 1 | Saline (0.15% sterile sodium chloride, 400 µl) |
| 2 | IGF-I (20 µg/d in 400 µl saline) |
| 3 | IGF-I (50 µg/d in 400 µl saline) |
| 4 | Anti-IGF-I antiserum (400 µl) |
| 5 | Anti-IGF-I antiserum (400 µl) pre-incubated for 1 h at room temperature with 20 µg/d IGF-I |

Mice were given good quality pelleted rodent feed and water *ad libitum* and were housed as five animals per cage (one from each treatment group). Daily treatments were administered subcutaneously as two equal aliquots at 9am and 4pm for seven days, starting when the mice were ten weeks old. Whole body weight was recorded daily at 9am prior to injection and tissues were dissected out and weighed at slaughter (by decapitation). The data were analysed by one-way analysis of variance.

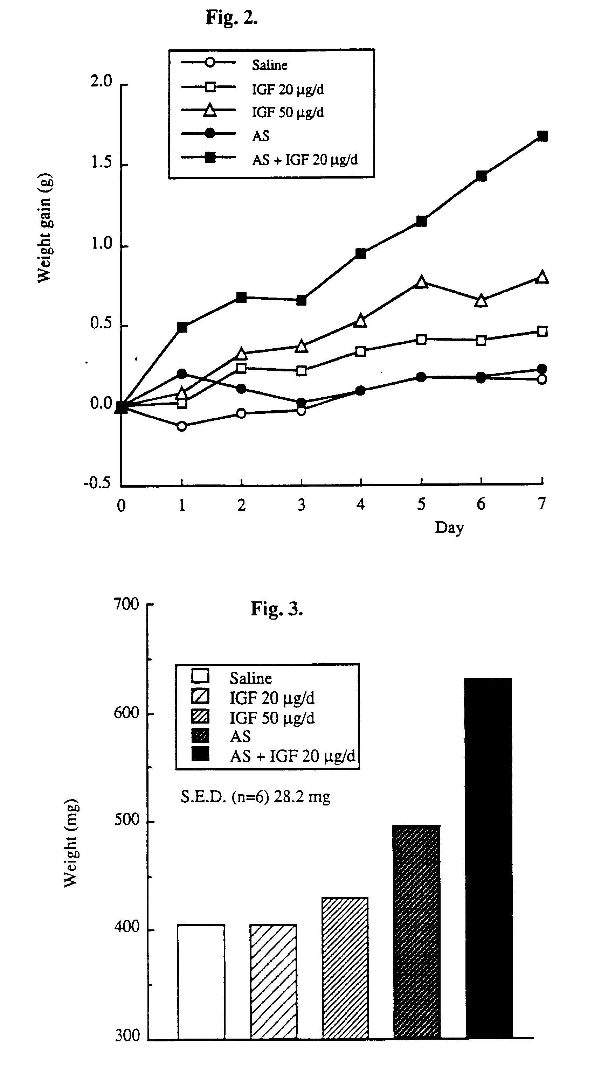

Figure 2 shows the whole body weight gain of the mice. Weight gain increased in a dose-dependent manner in response to IGF-I alone. Mice administered the anti-IGF-I antiserum in complex with 20 µg IGF-I per day exhibited at least a two-fold increase in daily gain compared with mice treated with 50 µg IGF-I alone (P<0.001). The data imply antiserum enhancement of IGF-I activity. Liver weights are illustrated in Figure 3. Injection of IGF-I alone did not change liver weight, whereas anti-IGF-I antiserum alone or in complex with IGF-I increased liver weight (P<0.05 and P<0.01, respectively) implying potentiation of endogenous and exogenous IGF-I action.

### Example 2: The effects of anti-IGF-I antiserum on growth in dwarf rats

Homozygous dwarf rats have a genetic lesion resulting in low (but not negligible) GH secretion and therefore reduced circulating IGF-I concentrations (about 25% of normal levels), resulting in a chronically reduced growth rate. Female homozygous dwarf rats were bred from a colony at the Institute of Animal Physiology and Genetics Research and eight-week old dwarf rats were randomly allocated to one of four treatment groups (n=6 per group):

| Group | Treatment |
|---|---|
| 1 | Non-immune serum (NIS, 3.0 ml/d) |
| 2 | Non-immune serum (3.0 ml/d) pre-incubated for 1 h at room temperature with 150 µg IGF-I/d |
| 3 | Anti-IGF-I antiserum (3.0 ml/d) |
| 4 | Anti-IGF-I antiserum (3.0 ml/d) pre-incubated for 1 h at room temperature with 150 µg IGF-I/d |

Non-immune serum was prepared from blood derived from normal control wether sheep (Poll Dorset). Daily treatments were administered subcutaneously as two equal aliquots at 9am and 4pm for seven days when the rats were nine weeks old. Whole body and tissue weights were recorded and the data analysed by two-way analysis of variance with IGF-I and antiserum as main treatment effects.

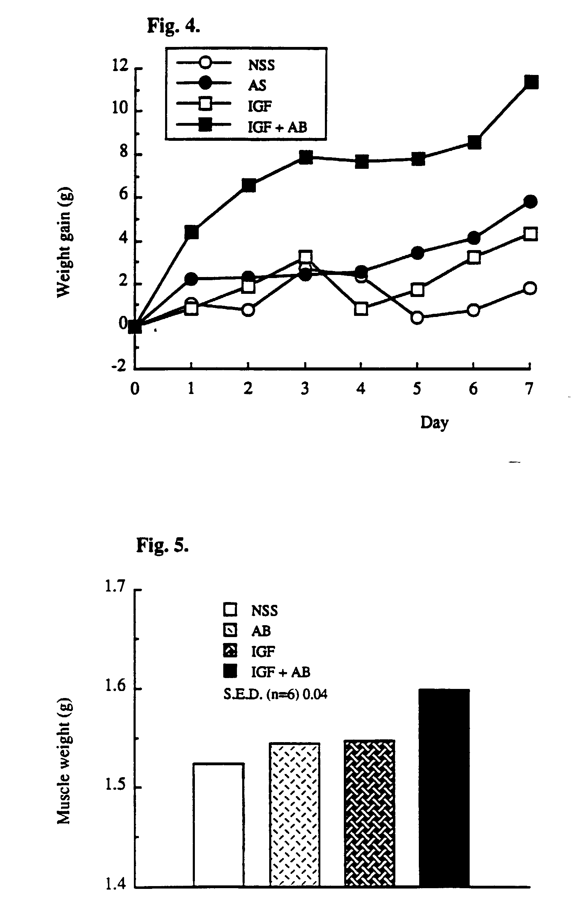

Figure 4 shows the whole-body weight gain of the rats. Rats treated with anti-IGF-I antiserum (anti-IGF-I) grew faster than those treated with non-immune serum. Daily gains (g/d) are summarised below:

| NIS | NIS +IGF | Anti-IGF-I | Anti-IGF +IGF | S.E.D. (n=6) | Main effects | |
|---|---|---|---|---|---|---|
| | | | | | IGF | Anti-IGF-I |
| 0.16 | 0.47 | 0.64 | 1.23 | 0.38 | NS | 0.032 |

IGF-I alone did not induce a significant increase in average daily gain whereas treatment with test antiserum did (P=0.032). The data imply that antiserum to IGF-I can enhance both endogenous and exogenous IGF-I.

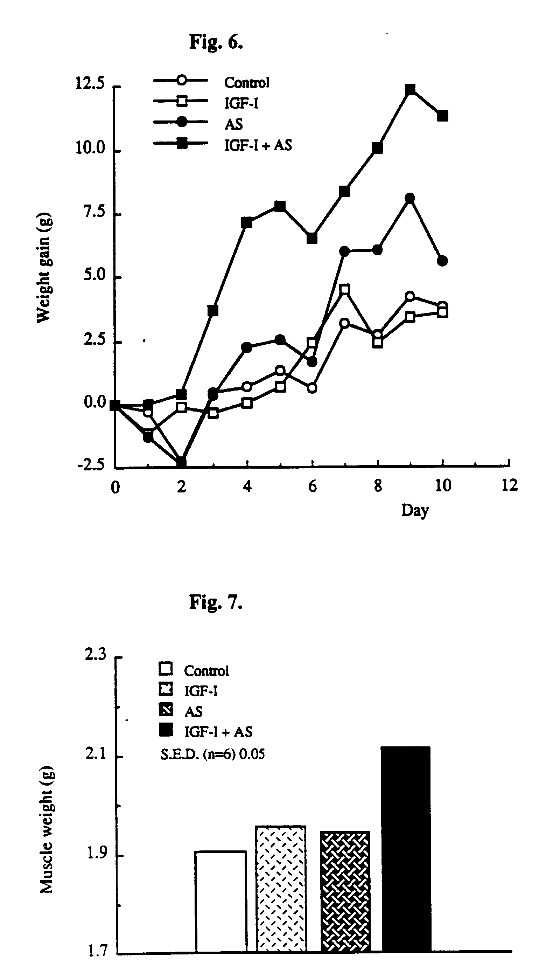

Figure 5 shows the muscle weights (gastrocnemius) dissected out at slaughter. No statistically significant changes were observed but the IGF-I antiserum complex tended to induce increased muscle weight.

### Example 3: The effects of semi-purified anti-IGF-I immunoglobulin on growth in dwarf rats

Semi-purified immunoglobulin was prepared from non-immune and anti-IGF-I antiserum by incubating the serum with saturated ammonium sulphate (0.666 x original serum volume) for 10 min at room temperature. The resultant precipitate was isolated by centrifugation at 10,000 g at 4°C for 10 min. The pellet was resuspended in distilled water to the original serum volume and re-mixed with fresh saturated ammonium sulphate (0.666 x serum volume). The immunoglobulin was isolated by centrifugation as before and the pellet resuspended in a minimum volume of PBS and dialysed extensively against PBS to remove ammonium sulphate. The immunoglobulin solution was then diluted to half of the original serum volume. The anti-IGF-I immunoglobulin preparation had a titre for IGF-I of approximately double that of the original serum when equivalent volumes were tested and the non-immune serum exhibited negligible IGF-I binding as determined by the ELISA assay.

Eight-week old female homozygous dwarf rats (as in Example 2) were allocated to one of four treatment groups:

| Group | Treatment |
|---|---|
| 1 | Semi-purified non-immune immunoglobulin (1.5 ml/d) |
| 2 | Semi-purified non-immune immunoglobulin (1.5 ml/d) pre-incubated for 1 h at room temperature with 150 µg IGF-I/d |
| 3 | Semi-purified anti-IGF-I immunoglobulin (1.5 ml/d) |
| 4 | Semi-purified anti-IGF-I immunoglobulin (1.5 ml/d) pre-incubated for 1 h at room temperature with 150 µg IGF-I/d |

Daily treatments were administered subcutaneously as two equal aliquots at 9am and 4pm for ten days when the rats were nine weeks old. Whole body and tissue weights were recorded and the data analysed by two-way analysis of variance with IGF-I and antiserum as main treatment effects.

Whole body weight gains are illustrated in Figure 6. Average daily gains were calculated for each group as g/d and are presented below. When main effects of IGF-I and antiserum were considered, IGF-I induced no significant growth stimulation, whereas anti-IGF-I immunoglobulin did (P=0.035). These data imply enhancement of exogenous and endogenous IGF-I activity.

The total weights of dissected leg muscles (gastrocnemius, plantaris, soleus) are presented in Figure 7. As main effects, both IGF-I (P=0.010) and anti-IGF-I antiserum (P=0.018) stimulated significant increases in muscle weight. These total changes were reflected in the individual muscle weights, given below.

| | NIS | NIS +IGF | Anti-IGF-I | Anti-IGF +IGF | S.E.D. (n=6) | Main effects: | |
|---|---|---|---|---|---|---|---|
| | | | | | | IGF | Anti-IGF-I |
| Whole body daily gain (g/d) | 0.47 | 0.51 | 0.86 | 1.35 | 0.38 | NS | 0.035 |
| Gastrocnemius weight (g) | 1.508 | 1.557 | 1.532 | 1.687 | 0.045 | 0.005 | 0.026 |
| Plantaris weight (g) | 0.285 | 0.280 | 0.295 | 0.303 | 0.010 | NS | 0.044 |
| Soleus weight (g) | 0.113 | 0.118 | 0.117 | 0.124 | 0.006 | NS | NS |

### Example 4 - Peptide scanning

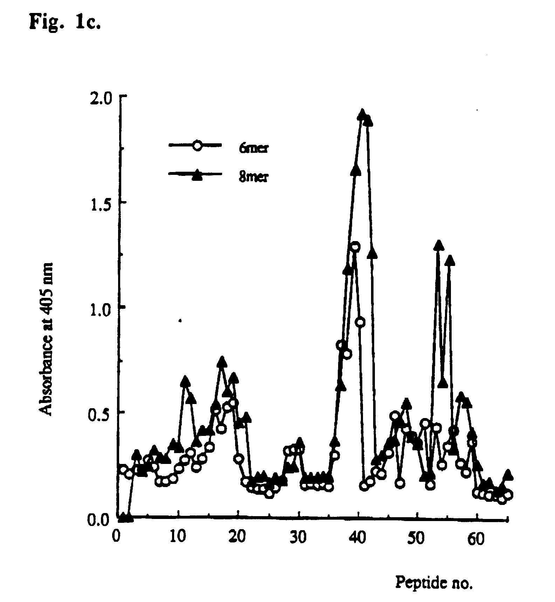

This example shows the results of peptide scanning the enhancing polyclonal anti-IGF-I antibody from sheep 50 (see "Antibody Production", immediately before Example 1). The protocol used 6mers and 8mers along the IGF-I sequence and identified the following residues as possible enhancing epitopes: 11-17, 18-21, 36-44 (very strong), 45-53 and 54-60. These are illustrated in Figure 1c. The methodology used was exactly as described in the manufacturer's instructions (Cambridge Research Biochemicals, Northwich, Cheshire: Epitope Scanning Kit) .

### Example 5 - Effects of antibodies to specific peptide regions of IGF-I

Antibodies raised in sheep to the following peptide regions have enhanced IGF-I activity, at least in terms of weight gain in dwarf rats: 1-17, 22-37, 59-70; antibodies raised to peptide 49-58 appear to have inhibited this particular IGF-I action. The method followed was as: Lachmann, P.J., Strangeways, L., Vyakarnam, A & Evan, G.I. (1986) "Raising antibodies by coupling peptides to PPD and immunizing BCG-sensitized animals", In Synthetic Peptides as Antigens, Ciba Foundation Symposium, 119, pp 25-40. John Wiley & Sons: London and New York. (PPD=purified protein derivative of tuberculin and BCG=Bacillus Calmette-Guerin). The methodology used for the animal experiment is exactly as for Example 3 except 200 µg IGF-I per day were used, not 150 µg/day, and the experiment was for 7 days and analysis was by one-way analysis of variance. Treatment groups and weight gain at 7 days was as follows:

| Group | Treatment | Whole body wt. gain at 7 d (g) |
|---|---|---|
| Control | Semi-purified non-immune immunoglobulin (1.5 ml/day) | 5.43 |
| IGF-I | Semi-purified non-immune immunoglobulin (1.5 ml/day) pre-incubated for 1 h at room temperature with 200 µg IGF-I/day | 7.30 |
| IGF-I plus anti-1-17 | Semi-purified anti-1-17 immunoglobulin (1.5 ml/day) pre-incubated for 1 h at room temperature with 200 µg IGF-I/day | 11.34* |
| IGF-I plus anti-49-58 | Semi-purified anti-49-58 immunoglobulin (1.5 ml/day) pre-incubated for 1 h at room temperature with 200 µg IGF-I/day | 4.83 |
| IGF-I plus anti-22-37 | Semi-purified anti-22-37 immunoglobulin (1.5 ml/day) pre-incubated for 1 h at room temperature with 200 µg IGF-I/day | 11.16* |
| IGF-I plus anti-59-70 | Semi-purified anti-59-70 immunoglobulin (1.5 ml/day) pre-incubated for 1 h at room temperature with 200 µg IGF-I/day | 9.54 |
| S.E.D. (n=7) | | 0.1675 |

| | | |
|---|---|---|
| * sig. diff from IGF-I plus non-immune immunoglobulin (P<0.05) | | |

### Example 6: Synthesis of Predicted Enhancing Epitopes and Production of Antibodies to these Peptide Sequences

### Peptide synthesis

The region of IGF-I which showed very strong binding to the enhancing anti-IGF-I antiserum was synthesised as two peptides using a Millipore/Biosearch 9500 peptide synthesiser (New Brunswick Scientific UK Ltd, Watford, England). The peptides were:

APQTGIVDC
PQTGIVDEC

The C residues were added for conjugation purposes.

### Peptide conjugation

The peptides were conjugated via the C residue to purified protein derivative of tuberculin (PPD, from Statens Seruminstitute, Copenhagen, Denmark) using sulphosuccinimidyl 4-(N-maleimidomethyl)-cyclohexane-1-carboxylate (sulpho-SMCC, Pierce Chemical Company, Rockford, USA). PPD elicits its own delayed hypersensitivity reaction in animals which have encountered tubercle bacillus. A 10 ml vial of PPD (1 mg/ml) was freeze-dried overnight and reconstituted with 2 ml glass distilled water. 2.5 mg sulpho-SMCC were added and the solids dissolved using an ultrasonic water bath. The pH of the solution was adjusted to 7.5 with 4 M NaOH and incubated at room temperature for 30 min. The solution was added to a 1.6 cm x 22 cm Ultrogel AcA 202 gel filtration column (IBF Biotechnics, Villeneuve-la-Garenne, France) and eluted at 4°C with 50 mM sodium phosphate buffer, pH 6.0. Activated PPD emerged at the exclusion volume of the column and unreacted sulpho-SMCC in later fractions; 10 mg peptide was pooled with activated PPD. After adjusting the pH to 7.0 with 5 M NaOH the solution was gassed with nitrogen and stored at room temperature overnight. The volume was adjusted to 10 ml using distilled water and stored at -70°C in 1 ml aliquots.

### Production of antibodies in sheep

Four sheep (Poll Dorset wethers aged one year) were injected with Bacillus Calmette-Guérin (BCG, reconstituted from freeze dried material with distilled water) as a 0.1 ml intradermal injection (equivalent to 8 x 10⁵ colony forming units).

One month after the BCG injections, 1 ml conjugated peptide was homogenised with 3 ml Freund's Incomplete Adjuvant and the sheep were injected with two by 0.75 ml emulsion intramuscularly plus two by 0.25 ml injections subcutaneously. Two sheep were immunised with each of the two peptides specified above. Boosts were carried out at 3 week intervals as described here except two by 0.5 ml injections were given intramuscularly and two by 0.5 ml injections subcutaneously. Serum was collected 10 days after each boost for antibody purification.

## Claims

1. An antibody or a derivative thereof, which is capable of binding to insulin-like growth factor-I (IGF-I) and which is capable of enhancing a biological activity of IGF-I.

2. An antibody as claimed in claim 1, which is a monoclonal antibody.

3. An antibody as claimed in claim 1, which is a humanised antibody.

4. An antibody as claimed in any one of claims 1 to 3, wherein the biological activity of IGF-I is an IGF-I-mediated anabolic action of growth hormone.

5. An antibody as claimed in any one of claims 1 to 4, which is capable of enhancing at least one of the following biological activities of IGF-I:
Increasing whole body and muscle growth rate in normal and hypopituitary animals;
Protection of body weight and nitrogen loss during catabolic states (such as fasting, nitrogen restriction, elevated corticosteroid levels and/or diabetes);
Kidney regeneration;
Nerve regeneration;
Hypoxia;
Wound healing;
Cardiac regeneration;
Cancer cachexia;
Angiogenesis;
Regeneration of the gastrointestinal tract;
Stimulation of mammary function;
IGF-I-dependent actions of GH (such as, usefulness in metabolic stress, age-related decreases in GH activity and adult GH deficiency);
Usefulness in maturity-onset diabetes; and/or
Usefulness in specific IGF-I deficiency.

6. An antibody as claimed in any one of claims 1 to 5, which binds to at least part of the region encompassing residues 1 to 17 of IGF-I.

7. An antibody or a derivative therefore which is capable of binding to insulin-like growth factor-I (IGF-I) and which is capable of enhancing a biological activity of IGF-I, for use in medicine.

8. An antigenic molecule which is capable, upon administration to a subject, of causing antibodies to be raised against a region encompassing at least four residues from residues 1 to 17, 18 to 21, 22 to 37, 36 to 44, 45 to 53, 54 to 60, or 59 to 70, of IGF-I, wherein the antibodies are capable of binding to insulin-like growth factor-I (IGF-I) and of enhancing a biological activity of IGF-I.

9. An antigenic molecule as claimed in claim 8, which comprises at least four residues from residues 36 to 44 of IGF-I.

10. A pharmaceutical formulation comprising a molecule as claimed in any one of claims 1 to 9 and a pharmaceutically acceptable carrier.

11. A product comprising IGF-I and a molecule as claimed in any one of claims 1 to 9 for simultaneous, separate or sequential use in therapy in which IGF-I is useful.

12. The use of a molecule as claimed in any one of claims 1 to 9 in the manufacture of a medicament for treating or preventing conditions in which IGF-I is useful.

## Patentansprüche

1. Antikörper oder ein Derivat davon, der oder das in der Lage ist, sich an einen insulinähnlichen Wachstumsfaktor-I (IGF-I) anzubinden, und der oder das in der Lage ist, eine biologische Tätigkeit des IGF-I zu verstärken.

2. Antikörper nach Anspruch 1, bei dem es sich um einen monoklonalen Antikörper handelt.

3. Antikörper nach Anspruch 1, bei dem es sich um einen humanisierten Antikörper handelt.

4. Antikörper nach einem der Ansprüche 1 bis 3, wobei es sich bei der biologischen Aktivität von IGF-I um eine vom IGF-I vermittelte anabolische Wirkung von Wachstumshormon handelt.

5. Antikörper nach einem der Ansprüche 1 bis 4, der in der Lage ist, mindestens eine der folgenden biologischen Aktivitäten von IGF-I zu verstärken:
Beschleunigung der Wachstumsrate des ganzen Körpers und der Muskeln bei normalen Tieren und solchen mit Hypophysenunterfunktion;
Schutz des Körpergewichts und Schutz vor Stickstoffverlust bei katabolischen Zuständen (wie z.B. beim Fasten, bei Stickstoffrestriktion, bei erhöhtem Nebennierenrindenhormonspiegel und/oder Diabetes);
Nierenregeneration;
Nervenregeneration;
Hypoxie;
Wundheilung;
Herzregeneration;
Krebskachexie;
Blutgefäßentwicklung;
Regeneration des gastrointestinalen Trakts;
Anregung der Brustdrüsenfunktion;
IGF-I-abhängige Wirkungen von Wachstumshormon (wie zum Beispiel positive Wirkung bei Stoffwechselbelastungen, altersbedingte Abnahme der Wachstumshormonaktivität und Wachstumshormonmangel bei Erwachsenen);
positive Wirkung bei Altersdiabetes; und/oder
positive Wirkung bei spezifischem IFG-I-Mangel.

6. Antikörper nach einem der Ansprüche 1 bis 5, der sich an mindestens einen Teil der Region anbindet, welche die Reste 1 bis 17 von IGF-I umfaßt.

7. Antikörper oder ein Derivat davon für die Verwendung zu medizinischen Zwecken, der in der Lage ist, sich an einen insulinähnlichen Wachstumsfaktor-I (IGF-I) anzubinden, und der in der Lage ist, eine biologische Tätigkeit von IGF-I zu verstärken.

8. Antigen-Molekül, das bei Verabreichung an ein Subjekt in der Lage ist, die Bildung von Antikörper gegen eine Region zu verursachen, welche mindestens vier Reste aus den Resten 1 bis 17, 18 bis 21, 22 bis 37, 36 bis 44, 45 bis 53, 54 bis 60, oder 59 bis 70 von IGF-I umfaßt, wobei die Antikörper in der Lage sind, sich an den insulinähnlichen Wachstumsfaktor-I (IGF-I) zu binden und eine biologische Aktivität von IGF-I zu fördern.

9. Antigen-Molekül nach Anspruch 8, welches mindestens vier Reste aus den Resten 36 bis 44 von IGF-I umfaßt.

10. Pharmazeutische Formulierung, umfassend ein Molekül nach einem der Ansprüche 1 bis 9, und einen pharmazeutisch akzeptablen Träger.

11. Produkt, umfassend IGF-I und ein Molekül nach einem der Ansprüche 1 bis 9, für die gleichzeitige, getrennte oder aufeinanderfolgende Anwendung in einer Therapie, bei der IGF-I von Nutzen ist.

12. Verwendung eines Moleküls nach einem der Ansprüche 1 bis 9 bei der Herstellung eines Medikamentes zur Behandlung von oder Vorbeugung gegen Leiden, bei denen IGF-I von Nutzen ist.

## Revendications

1. Anticorps ou dérivé de celui-ci, qui est capable de se lier à un facteur de croissance I analogue à l'insuline (IGF-I) et qui est capable de renforcer une activité biologique de l'IGF-I.

2. Anticorps selon la revendication 1, qui est un anticorps monoclonal.

3. Anticorps selon la revendication 1, qui est un anticorps humanisé.

4. Anticorps selon l'une quelconque des revendications 1 à 3, dans lequel l'activité biologique de l'IGF-I est une action anabolique d'une hormone de croissance médiée par l'IGF-I.

5. Anticorps selon l'une quelconque des revendications 1 à 4, qui est capable de renforcer au moins l'une des activités biologiques suivantes de l'IGF-I :
augmentation du taux de croissance de la masse corporelle entière et de la masse musculaire chez des animaux normaux et hypopituitaires,
protection du poids du corps et de la perte d'azote au cours d'états cataboliques (tels que le jeûne, la restriction d'azote, des niveaux élevés de corticostéroïdes et/ou le diabète),
régénération des reins,
régénération nerveuse,
hypoxie,
cicatrisation de blessures,
régénération cardiaque,
cachexie due au cancer,
angiogenèse,
régénération des voies gastro-intestinales,
stimulation de la fonction mammaire,
actions du GH fonction de l'IGF-I (telles que l'utilité dans le stress métabolique, les réductions d'activité du GH dues à l'âge et la déficience de GH chez les adultes),
utilité dans le diabète en début de maturité, et/ou
utilité dans la déficience spécifique d'IGF-I.

6. Anticorps selon l'une quelconque des revendications 1 à 5, qui se lie à au moins une partie de la région comprenant les résidus 1 à 17 de l'IGF-I.

7. Anticorps ou dérivé de celui-ci, qui est capable de se lier au facteur de croissance I analogue à l'insuline (IGF-I) et qui est capable de renforcer une activité biologique de l'IGF-I, destiné à un usage en médecine.

8. Molécule antigénique qui est capable, lorsqu'elle est administrée à un sujet, de provoquer la formation d'anticorps contre une région comprenant au moins quatre résidus allant des résidus 1 à 17, 18 à 21, 22 à 37, 36 à 44, 45 à 53, 54 à 60 ou 59 à 70, de l'IGF-I, les anticorps étant capables de se lier à un facteur de croissance I analogue à l'insuline (IGF-I) et de renforcer une activité biologique de l'IGF-I.

9. Molécule antigénique selon la revendication 8, qui comprend au moins quatre résidus allant des résidus 36 à 44 de l'IGF-I.

10. Formulation pharmaceutique comprenant une molécule selon l'une quelconque des revendications 1 à 9 et un véhicule acceptable au plan pharmaceutique.

11. Produit comprenant de l'IGF-I et une molécule selon l'une quelconque des revendications 1 à 9 pour un usage simultané, séparé ou séquentiel en thérapie dans laquelle l'IGF-I est utile.

12. Utilisation d'une molécule selon l'une quelconque des revendications 1 à 9 dans la fabrication d'un médicament pour traiter ou prévenir des états dans lesquels l'IGF-I est utile.
